# EUROPEAN PATENT SPECIFICATION

(11) **EP 1 711 150 B1**
(45) Date of publication and mention of the grant of the patent: **28.05.2008**
(21) Application number: 04809063.3
(22) Date of filing: 16.12.2004
(51) Int. Cl.: A61F 13/20, A61F 13/34, A61F 13/26, A61F 13/28

(54) **A TAMPON**
TAMPON
TAMPON HYGIENIQUE

(30) Priority: 30.12.2003 SE 0303559
(43) Date of publication of application: 18.10.2006
(73) Proprietor: SCA Hygiene Products AB, 405 03 Göteborg (SE)
(72) Inventor: HAGBERG, Katarina, S-436 55 Hovas (SE); STRIDFELDT, Chatrine, S-436 58 Hovas (SE); DREVIK, Solgun, S-435 35 Mölnlycke (SE)
(74) Representative: Romare, Laila Anette
(86) International application number: PCT/SE2004/001889
(87) International publication number: WO 2005/063161

(56) References cited:
- WO-A2-00/54714
- US-A- 4 211 225
- US-A1- 2003 028 177

## Description

### TECHNICAL AREA:

The invention pertains to a tampon comprising an absorption body having generally elongate shape and having an insertion end and a withdrawal end and comprising a withdrawal string attached to the absorption body and extending from the withdrawal end.

### BACKGROUND OF THE INVENTION:

Menstrual tampons for intra-vaginal use have been known and used for a very long time. Since tampons are worn internally, they are considered to be discrete and mostly very comfortable to wear, lacking the occluding plastic backing of external protection devices such as sanitary napkins. However, during days of light menstrual flow or when changing the tampon after a short period of use, removal of the tampon may cause severe discomfort or even pain. This is due to the tampon absorbing menstrual discharge as well as humidity from the mucous membranes on the vaginal wall. When the tampon is being removed, it tends to adhere to the vaginal wall giving rise to increased friction and making it hard to extract. Moreover, the dried-out mucous membranes make it almost impossible or at least very difficult and uncomfortable to insert a new tampon to replace the one that has been removed.

The problems experienced when changing tampons that have not been used to their full capacity lead to users wearing tampons for longer periods of time than recommended. This is highly undesirable in that it increases the risk of bacterial infections.

In order to facilitate removal of a used tampon, it has been suggested to treat the surface of the tampon with lubricating agents decreasing the friction between the tampon and the vaginal wall. Such treatment suffers from several drawbacks. Accordingly, lubricating agents are mostly hydrophobic materials that may migrate into the absorbent material and restrict fluid flow into the absorbent body of the tampon. Moreover, the use of lubricating agents complicates the production process and puts particular demands on packaging and handling of the tampons. In addition, it is desirable that the friction between the tampon and the vaginal wall remains sufficiently high during use of the tampon so that the tampon is kept securely in place and is not displaced for instance when the user sneezes or coughs.

Hence, there exists a need for a vaginal tampon that can be easily and comfortably removed even when only partially saturated by menstrual fluid.

### DESCRIPTION OF THE INVENTION:

In accordance with the invention, there is provided tampon comprising an absorption body having generally elongate shape and having an insertion end and a withdrawal end and comprising a withdrawal string attached to the absorption body and extending from the withdrawal end. The tampon is primarily distinguished in that it comprises a withdrawal aid being attached to and extending from the insertion end and being arranged to be movable between a first position in which the withdrawal aid at least partly covers the absorption body and a second position in which the withdrawal aid extends away from the absorption body, and the withdrawal aid is attached to the surface of the absorption body by means of a liquid soluble binder.

The insertion end of the tampon is that part of the tampon, including the tip of the tampon that is first introduced in the vagina. The withdrawal end of the tampon is the leading end when the tampon is withdrawn after use.

During use of the tampon in accordance with the invention, the withdrawal aid acts as a spacing means, creating a distance between the mucous membranes on the user's vaginal wall and the absorption body. When the tampon is being pulled out after use, the withdrawal aid will move from the first position to the second position by inverting or peeling away from the absorption body. In this manner, removal of the tampon is greatly facilitated and chafing and abrasion of the mucous membranes is reduced. The collapsed, inverted withdrawal aid is easy to pull away from the vaginal wall without causing any pain or other discomfort.

The tampon according to the invention has preferably essentially cylindrical shape and can be enclosed in a liquid permeable cover in order to minimise linting from the absorption body.

According to one embodiment of the invention, the withdrawal aid consists of an extension of the liquid-permeable cover. The withdrawal aid can be of a material that is identical to the rest of the liquid-permeable cover. Alternatively, the extension may have been treated by being perforated in order to enhance liquid permeability. It is also possible to treat the extension in order to increase the friction on the surface that is intended to contact the vaginal wall during insertion. Such treatment can be made by physical modification of the extension, such as by embossing or by adding a friction enhancing agent to the side of the extension contacting the vaginal wall during insertion and the initial stage of withdrawal. By increasing the friction between the vaginal wall and the withdrawal aid in relation to the friction between the withdrawal aid and the tampon cover, it is ascertained that sliding motion will primarily take place between the cover and the withdrawal aid and not between the withdrawal aid and the vaginal wall.

The withdrawal aid in accordance with the invention may comprise a perforated nonwoven material.

According to one embodiment of the invention, the withdrawal aid comprises a plurality of flexible elongate elements.

The withdrawal aid preferably extends at least one third of the distance between the insertion end and the withdrawal end of the tampon when the withdrawal aid is in the first position.

The withdrawal aid may comprise a nonwoven material, a perforated plastic film, a flexible open-celled foam or a net.

In order to facilitate sliding motion between the absorption body and the withdrawal aid a friction reducing agent may be applied between the withdrawal aid and the absorption body when the withdrawal aid is in the first position. The friction reducing agent may also act as a lubricating means for lubricating the mucous membranes in the user's vagina.

The friction reducing agent may be chosen among pectin, hyaluronic acid, glycerides, waxes such as silicone waxes, plant waxes or paraffin wax. However, these compounds are only a few examples of a large variety of substances that can be used as friction reducing agents.

The withdrawal aid may be provided with friction enhancing means on a surface thereof that is facing outwardly when the withdrawal aid is in the first position. The friction enhancing means may be provided mechanically by physically modifying the material in the withdrawal aid such as by perforation or embossing or by applying a friction-enhancing agent such as a coating of wax or an elastomeric polymer.

In order to maintain the withdrawal aid in position at least during insertion and the initial period of use, the withdrawal aid is attached to the surface of the absorption body by means of a liquid soluble binder. Suitable binders are polyvinyl alcohol (PVA), polyethylene oxide (PEO), polyvinyl pyrrolidone (PVP) or cellulose derivatives such as Hydroxypropyl methylcellulose (HPMC), Hydroxypropyl cellulose (HPC) or Hydroxyethyl cellulose (HEC). As the tampon absorbs liquid and becomes wet, the adhesive dissolves, allowing the withdrawal aid to come away from the absorption body upon removal of the tampon.

The tampon may also comprise an active substance chosen among odour controlling agents, perfumes, lactic acid producing organisms, pain control agents, sedatives or mixtures thereof.

Such active substances may be placed on the withdrawal aid, between the withdrawal aid and an inner cover on the tampon's absorption body or between the withdrawal aid and the absorption body. An active substance may also be placed in the absorption body. Combinations of active substances and their arrangement in the tampon may be employed.

### SHORT DESCRIPTION OF FIGURES:

The invention will in the following be described in greater detail, with reference to the figures that are shown on the appended drawings. In the drawings:
- Fig. 1: shows a tampon in accordance with a first embodiment of the invention;
- Fig. 2: shows the tampon in Fig. 1 during an initial stage of withdrawal;
- Fig. 3: shows the tampon in Figs. 1 and 2 during a final stage of withdrawal;
- Fig. 4: shows a tampon in accordance with a second embodiment of the invention;
- Fig. 5: shows the tampon in Fig. 3 during withdrawal;
- Fig. 6: shows a tampon in accordance with a third embodiment of the invention;
- Fig. 7: shows a tampon in accordance with a fourth embodiment of the invention; and
- Fig. 8: shows a tampon in accordance with a fifth embodiment of the invention.

### DETAILED DESCRIPTION OF EMBODIMENTS:

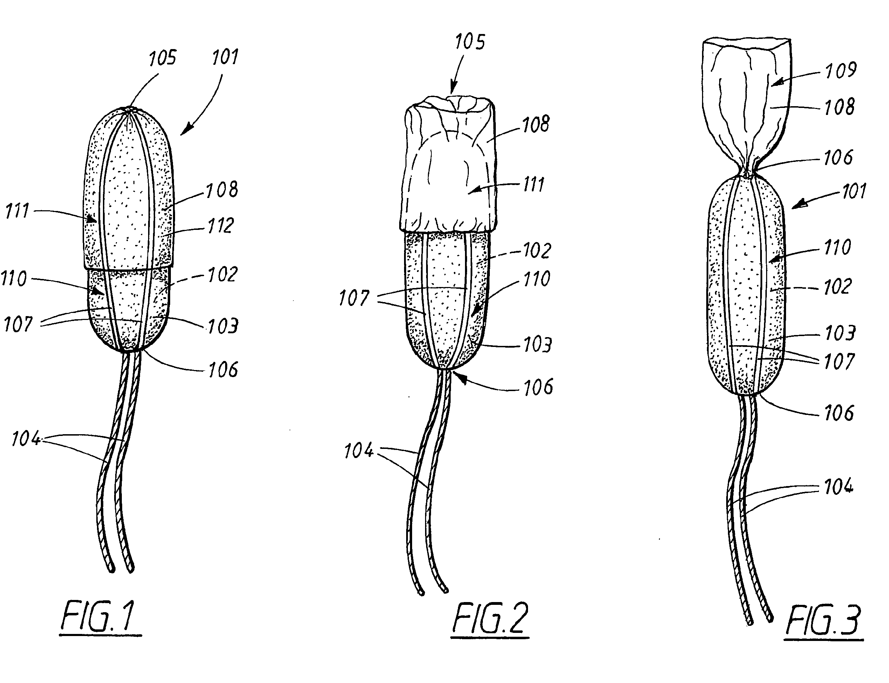

The tampon 101 shown in Figs. 1-3 comprises an absorption body 102 enclosed in a liquid permeable cover 103 and provided with two withdrawal strings 104. The tampon 101 has an elongate generally cylindrical shape with an insertion end 105 and a withdrawal end 106. The withdrawal strings 104 protrude from the withdrawal end 106.

The absorption body 102 is preferably a mass of absorbent fibres that has been compressed into a roughly cylindrical shape. As is common in the art and as shown in the Figs., the tampon is provided with longitudinally extending compressed grooves 107 that aid liquid distribution along the length of the tampon. Suitable absorbent materials for the absorption body 102 are cellulose fibres such as rayon, cotton and cellulose fluff pulp. The absorption body 102 may also comprise a binder such as thermoplastic fibres. Polymeric gelling materials, also known as superabsorbents, can be used as well as bacteria inhibiting agents.

The cover 103 may be any suitable non-abrasive liquid permeable material. Preferably, the cover 103 is a nonwoven material which may be a spunbonded, carded or spunlaced web made of polypropylene, polyethylene, viscose, bicomponent fibers or any other suitable fibrous material. However, perforated plastic films, cast or knitted nettings or similar porous materials may also be used.

The withdrawal strings 104 are made from a material having high tensile strength and are firmly attached to the tampon body by being wound internally in the absorption body 102 or by being welded, glued or sewn to the absorption body 102 and/or to the liquid permeable cover 103.

The cover 103 has an extension 108 at the withdrawal end 106 of the tampon 101. The tampon is delivered to the user with the extension 108 of the cover 103 folded over the absorption body 102 in a manner to create a two-layer covering at the insertion end 105 of the tampon 101. In the embodiment shown in Figs. 1-3, the extension 108 of the cover 103 reaches down from the tip of the tampon at the insertion end along approximately two thirds of the length of the absorption body 102. However, in alternative embodiments, the extension 108 may cover a longer or shorter distance along the absorption body 102 or may extend all the way to the withdrawal end 106.

The extension 108 has a first surface 109 which is an inner surface facing and overlying the outer surface 110 of the cover 103 enclosing the absorption body 102 when the tampon is in its insertion state, as shown in Fig. 1. An opposite, second surface 111 faces away from the outer surface 110 of the cover 103 and constitutes an outer surface 111 of the extension 108 when the tampon is in the insertion state.

The cover material is preferably chosen to provide a low friction between the first surface 109 of the extension 108 and the outer surface 110 of the cover 103. When, as in Figs. 1-3, the extension is merely a continuation of the cover material this can be achieved by choosing a material having different friction characteristics on different sides. It is also possible to use friction-reducing agents such as pectin, hyaluronic acid, glycerides, waxes such as silicon waxes, plant waxes or paraffin wax between the layers of cover material. However, if such friction reducing agents are employed, it is important to ascertain that they do not impede liquid flow into the absorption body 102.

It could be advantageous to use a lubricating means as a friction reducing agent. When the tampon is extricated, the extension 108 of the cover 103 will become inverted. This implies that the first surface 109 of the extension 108 will become exposed to the user's vaginal wall. The lubricating means facilitates the final stage of the withdrawal and can provide some lubrication of the mucous membranes in the vagina, thus facilitating insertion of a new tampon.

It is also possible to include active substances other than lubricating or friction-reducing agents in the tampon. Such substances can be positioned on the outer surface of the tampon and act in the same manner as in a conventional tampon. Alternatively, the active substance(s) may be placed between layers of covering material or between an outer invertible cover and the absorbent body of the tampon. By placing an active substance beneath an outer cover, it is possible to obtain a controlled release of the active substance. By choosing the material of the outer cover in a suitable way, the release of the substance may be controlled in such a way that the release lasts for a longer time during use than if it is just placed on an outside surface of the tampon. There is also a possibility to keep the active substance immobilised until withdrawal of the tampon. The active substance may be encapsulated until the tampon is withdrawn and the encapsulation broken by tensile forces and/or frictional forces. The active substance may be chosen among one or more of odour controlling agents, perfumes, lactic acid producing organisms, pain control agents, sedatives or any desired active substance. In the case of perfumes or odour control agents, the effect may be advantageous for the user as well as for prohibiting unwanted odour from the used and discarded tampon.

When extricating the tampon 101 after use, the user pulls at one or both of the withdrawal strings 104. The pulling action causes the absorption body 102 to move in the direction of the applied force. In doing so, the first surface 109 of the extension 108 starts to slide against the outer surface 110 of the cover 103. Since the friction between the two layers of cover material 103, 108 is less than the friction between the second surface 111 of the extension 108 and the user's vaginal wall, the second surface 111 of the extension 108 will cling to the vaginal wall. In this manner, the pulling force will also cause the extension 108 to gradually invert, as shown in Fig. 2. Finally, as is shown in Fig. 3, the extension 108 is completely inverted, forming an empty, collapsed, trailing portion of flexible material that will readily detach from the vaginal wall without causing the user any discomfort.

Hence, during withdrawal of the tampon 101, the extension 108 of the cover material acts as a removal aid 112 minimising the risk of causing abrasion or other friction-induced discomforts that may otherwise arise when a tampon is being withdrawn.

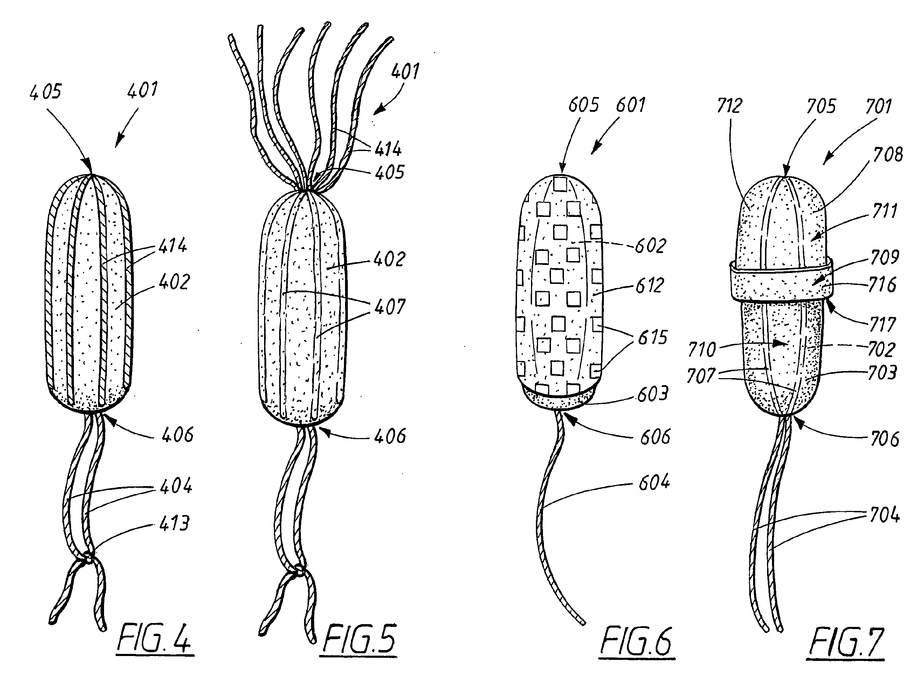

The tampon 401 shown in Figs. 4 and 5 is similar to that in Figs. 1-3. Accordingly, the tampon in Figs. 4 and 5 has a generally cylindrical absorption body 402 with compressed, longitudinally extending grooves 407 and is provided with a withdrawal string loop formed by joining two string ends in a knot 413.

In the Fig. 4 embodiment, the tampon 401 is shown without a liquid-permeable cover. However, such a cover can be used if desired. When the tampon has no cover, it is important that the absorbent material has high integrity so that it does not fall apart or shed fibres or particles during use. Usually such tampons are made from staple length fibres, such as carded rayon fibres.

The tampon 401 in Figs. 4 and 5 has a withdrawal aid 412 in the form of a plurality of flexible elongate elements 414. The elongate elements are strings, bands or fibres that are attached to the insertion end 405 of the absorption body 402. When the tampon 401 is in the insertion state, the elongate elements 414 extend from the insertion end 405 towards the withdrawal end 406 along the surface of the absorption body 402.

The insertion aid 412 in Figs. 4 and 5 acts in much the same way as the insertion aid 112 in Figs. 1-3 when the tampon is being withdrawn. Hence, during withdrawal, the elongate elements 414 will cling to the vaginal wall and will gradually be peeled away from the absorption body 402 until the configuration in Fig. 5 is achieved. The elongate elements 414 serve as spacing means between the vaginal wall and the absorption body 402 and prevent the absorption material from sticking to the vaginal wall. In this manner, friction between the vaginal wall and the absorption body 402 is reduced. Moreover, by minimising direct contact between the vaginal wall and the absorption material, shedding of fibres and particles from the absorption body 402 is also reduced. This is, of course, a particular benefit for tampons having no outer cover separating the absorption body 402 from the vaginal wall.

In order to maintain the elongate elements 414 in position at least during insertion and the initial period of use, the elongate elements 414 are attached to the surface of the absorption body 402 by means of a liquid soluble binder. Suitable binders are polyvinyl alcohol (PVA), polyethylene oxide (PEO), polyvinyl pyrrolidone (PVP) or cellulose derivatives such as hydroxypropyl methylcellulose (HPMC), hydroxypropyl cellulose (HPC) or hydroxyethyl cellulose (HEC). As the tampon absorbs liquid and becomes wet, the adhesive will dissolve, allowing the elongate elements to come away from the absorption body 402 upon removal of the tampon.

The elongate elements 414 can be non-absorbent, serving only as spacing means and friction reducing means. Alternatively, the elongate elements may be more or less absorbent. Preferably, the elongate elements 414 have a high wicking ability so that they contribute to disperse liquid along the absorption body 402. Suitable absorbent and/or wicking materials are cotton, rayon, polyurethane foam, profiled fibres having external capillaries, etc.

The embodiment shown in Fig. 6 is similar to that in Figs. 1-3 but differs in that the withdrawal aid 612 is a material having relatively large holes or perforations 615 and in that the withdrawal aid 612 extends all the way from the insertion end 605 to the withdrawal end 606 when the tampon is in the insertion state. The withdrawal aid 612 can be an extension of a cover 603 enclosing the absorption body 602 or can be made from a separate and different material. The withdrawal aid 612 is preferably made from a perforated nonwoven material, a perforated plastic film or a large-mesh net. In order to enhance the spacing capability of the withdrawal aid 612, a material having relatively coarse fibres or strands can be chosen. A good spacing effect is also obtained with a three-dimensionally formed perforated material.

By providing the withdrawal aid 612 with holes or perforations 615, fluid flow into the absorption body 602 is facilitated.

A further embodiment of the invention is shown in Fig. 7. The construction of the tampon in Fig. 7 is identical to that in Figs. 1-3, with the only exception that an edge portion 716 of the withdrawal aid 712 is folded back towards the insertion end 705 when the tampon is in the insertion state. The folded-back edge portion 716 is preferably fixed in the folded position with a liquid-soluble adhesive so that the edge portion 716 remains folded during insertion of the tampon 701.

The embodiment in Fig. 7 possibly offers even less frictional engagement between the tampon 701 and the user's vaginal wall than the embodiment in Figs. 1-3. During withdrawal of the tampon, the extension 708 of the cover will start to unfold from the fold 717 at the lower end of the folded edge portion 716.

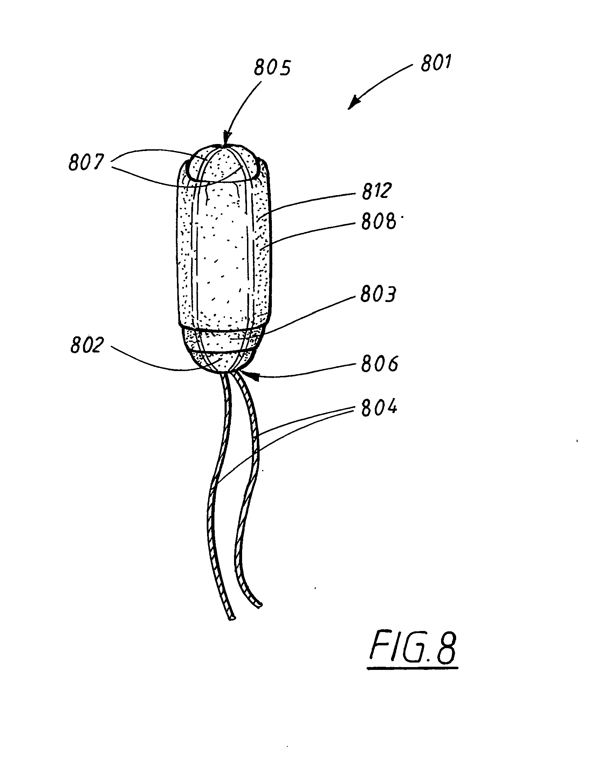

The tampon 801 shown in Fig. 8 is very similar to that shown in Figs. 1-3 and comprises an absorption body 802 having a liquid permeable cover 803 and being provided with two withdrawal strings 804. The tampon 801 has an elongate generally cylindrical shape with slightly rounded ends 805, 806. The withdrawal strings 104 protrude from the withdrawal end 806.

The tampon in Fig. 8 differs from that in Figs. 1-3 in that the liquid permeable cover 803 does not reach all the way to the very ends 805, 806 of the tampon. Instead, the liquid permeable cover 803 is applied as a girdle around the straight cylindrical portion of the tampon. The tampon 801 has an insertion aid 812 constituted by an extension 808 of the cover that is folded back over the cover 803 in the same general manner as described in connection with the cover extension 108 in Figs. 1-3. The fold is positioned at the insertion end 805, slightly below the tip of the tampon.

The tampon in Fig. 8 functions in the same manner as the tampon shown in Figs. 1-3, meaning that the extension 808 of the cover will invert as the tampon 801 is withdrawn after use.

## Claims

1. A tampon comprising an absorption body (102) having generally elongate shape and having an insertion end (105) and a withdrawal end (106) and comprising a withdrawal string (104) attached to the absorption body (102) and extending from the withdrawal end (106), wherein the tampon comprises a withdrawal aid (112) being attached to and extending from the insertion end (105) and being arranged to be movable between a first position in which the withdrawal aid (112) at least partly covers the absorption body (102) and a second position in which the withdrawal aid (112) extends away from the absorption body (102),
**characterized in**
**that** the withdrawal aid (412) is attached to the surface of the absorption body (402) by means of a liquid soluble binder.

2. A tampon according to claim 1, wherein the absorption body (102) is essentially cylindrical.

3. A tampon according to claim 1 or 2, wherein the absorption body (102) is surrounded by a liquid permeable cover (103).

4. A tampon according to claim 3, wherein the withdrawal aid (112) consists of an extension (108) of the liquid-permeable cover (103).

5. A tampon according to any one of claims 1-4, wherein the withdrawal aid (112) comprises a perforated nonwoven material.

6. A tampon according to any one of claims 1-3, wherein the withdrawal aid (412) comprises a plurality of flexible elongate elements (414).

7. A tampon according to any one of the preceding claims, wherein the withdrawal aid (112) extends at least one third of the distance between the insertion end (105) and the withdrawal end (106) when in the first position.

8. A tampon according to any one of the preceding claims, wherein the withdrawal aid (112) comprises a nonwoven material, a perforated plastic film or a net.

9. A tampon according to any one of the preceding claims, wherein a friction reducing agent is applied between the withdrawal aid (112) and the absorption body (902) when the withdrawal aid (112) is in the first position.

10. A tampon according to claim 10, wherein the friction-reducing agent is chosen among pectin hyaluronic acids, glycerides, waxes such as silicone waxes, plant waxes or paraffin wax.

11. A tampon according to any one of the preceding claims, wherein the withdrawal aid (112) is provided with friction-enhancing means on a surface thereof which is facing outwardly when the withdrawal aid is in the first position.

12. A tampon according to any one of the preceding claims, wherein the liquid soluble binder is chosen among polyvinyl alcohol (PVA), polyethylene oxide (PEO), polyvinyl pyrrolidone (PVP) or cellulose derivatives such as hydroxypropyl methylcellulose (HPMC), hydroxypropyl cellulose (HPC) or hydroxyethyl cellulose (HEC).

13. A tampon according to any one of the preceding claims, wherein the tampon comprises an active substance chosen among odour controlling agents, perfumes, lactic acid producing organisms, pain control agents, sedatives or mixtures thereof.

## Patentansprüche

1. Tampon umfassend einen Absorptionskörper (102) mit einer im Wesentlichen länglichen Form und einem Einführende (105) sowie einem Entnahmeende (106) und umfassend ein Entnahmeschnürchen (104), das an dem Absorptionskörper angebracht ist und sich von dem Entnahmeende 106 erstreckt, wobei der Tampon eine Entnahmehilfe (112) umfasst, die an dem Einführende (105) angebracht sich von diesem erstreckt sowie derart angeordnet ist, dass sie zwischen einer ersten Position, in der die Entnahmehilfe (112) den Absorptionskörper (102) wenigstens teilweise bedeckt und einer zweiten Position, in der sich die Entnahmehilfe (112) von dem Absorptionskörper weg erstreckt, bewegbar ist,
**dadurch gekennzeichnet, dass**
die Entnahmehilfe (412) durch ein flüssigkeitslösliches Bindemittel angebracht ist an der Oberfläche des Absorptionskörpers (402).

2. Tampon nach Anspruch 1, bei der der Absorptionskörper (102) im Wesentlichen zylindrisch ist.

3. Tampon nach Anspruch 1 oder 2, bei dem der Absorptionskörper 102 durch eine flüssigkeitsdurchlässige Hülle (103) umgeben ist.

4. Tampon nach Anspruch 3, bei der die Entnahmehilfe (112) aus einem Fortsatz (108) der flüssigkeitsdurchlässigen Hülle (108) besteht.

5. Tampon nach einem der Ansprüche 1-4, bei dem die Entnahmehilfe (112) einen perforierten Vliesstoff umfasst.

6. Tampon nach einem der Ansprüche 1-3, bei dem die Entnahmehilfe (412) mehrere flexible längliche Elemente (414) umfasst.

7. Tampon nach einem der vorstehenden Ansprüche, bei sich die Entnahmehilfe (112) wenigstens über ein Drittel des Abstands zwischen dem Einführende (105) und dem Entnahmeende (106) erstreckt, wenn sie sich in der ersten Position befindet.

8. Tampon nach einem der vorstehenden Ansprüche, bei dem die Entnahmehilfe (112) einen Vliesstoff, eine perforierte Kunststofffolie oder ein Netz umfasst.

9. Tampon nach einem der vorstehenden Ansprüche, bei dem ein die Reibung reduzierendes Mittel zwischen der Entnahmehilfe (112) und dem Absorptionskörper (102) aufgebracht wird, wenn sich die Entnahmehilfe (112) in der ersten Position befindet.

10. Tampon nach Anspruch 10, bei der das die Reibung reduzierende Mittel aus Pektinhyaluronsäure, Glyceriden, Wachsen, wie beispielsweise Silikonwachsen, Pflanzenwachsen oder Parafinwachs gewählt ist.

11. Tampon nach einem der vorstehenden Ansprüche, bei dem die Entnahmehilfe (112) mit einer die Reibung erhöhenden Einrichtung auf einer Oberfläche davon versehen ist, die nach außen weist, wenn sich die Entnahmehilfe in der ersten Position befindet.

12. Tampon nach einem der vorstehenden Ansprüche, bei der das flüssigkeitslösliche Bindemittel gewählt ist aus Polyvinylalkohol (PVA), Polyethylenoxid (PEO), Polyvinylpyrrolidon (PVP) oder Cellulosederivaten, wie beispielsweise Hydroxpropylmethylcellulose (HPMC), Hydroxypropylcellulose (HPC) oder Hydroxyethylcellulose (HEC).

13. Tampon nach einem der vorstehenden Ansprüche, bei dem der Tampon eine aktive Substanz umfasst, gewählt aus Geruch kontrollierenden Elementen, Parfümen, Milchsäure produzierenden Organismen, Schmerzmitteln, Sedativen oder Mischungen davon.

## Revendications

1. Tampon comprenant un corps d'absorption (102) ayant une forme généralement allongée, et ayant une extrémité d'insertion (105) et une extrémité de retrait (106), et comprenant un cordon de retrait (104) fixé sur le corps d'absorption (102) et s'étendant à partir de l'extrémité de retrait (106), le tampon comprenant un auxiliaire de retrait (112) fixé sur l'extrémité d'insertion (105), et s'étendant à partir de celle-ci, et agencé pour être mobile entre une première position dans laquelle l'auxiliaire de retrait (112) recouvre au moins partiellement le corps d'absorption (102), et une seconde position dans laquelle l'auxiliaire de retrait (112) s'étend le long du corps d'absorption (102),
**caractérisé en ce que**
l'auxiliaire de retrait (412) est fixé sur la surface du corps d'absorption (402) par l'intermédiaire d'un liant soluble dans un liquide.

2. Tampon selon la revendication 1, dans lequel le corps d'absorption (102) est essentiellement cylindrique.

3. Tampon selon la revendication 1 ou 2, dans lequel le corps d'absorption (102) est entouré d'un recouvrement perméable aux liquides (103).

4. Tampon selon la revendication 3, dans lequel l'auxiliaire de retrait (112) est constitué d'un prolongement (108) du recouvrement perméable aux liquides (103).

5. Tampon selon l'une quelconque des revendications 1 à 4, dans lequel l'auxiliaire de retrait (112) comprend un matériau de non-tissé perforé.

6. Tampon selon l'une quelconque des revendications 1 à 3, dans lequel l'auxiliaire de retrait (412) comprend une pluralité d'éléments allongés souples (414).

7. Tampon selon l'une quelconque des revendications précédentes, dans lequel l'auxiliaire de retrait (112) s'étend sur au moins un tiers de la distance entre l'extrémité d'insertion (105) et l'extrémité de retrait (106) lorsqu'il est dans la première position.

8. Tampon selon l'une quelconque des revendications précédentes, dans lequel l'auxiliaire de retrait (112) comprend un matériau non-tissé, un film en matière plastique perforé ou un filet.

9. Tampon selon l'une quelconque des revendications précédentes, dans lequel un agent de réduction de frottement est appliqué entre l'auxiliaire de retrait (112) et le corps d'absorption (102) lorsque l'auxiliaire de retrait (112) est dans la première position.

10. Tampon selon la revendication 10, dans lequel l'agent de réduction de frottement est choisi parmi des acides hyaluroniques de pectine, des glycérides, des cires telles que des cires de silicone, des cires végétales ou de la paraffine.

11. Tampon selon l'une quelconque des revendications précédentes, dans lequel l'auxiliaire de retrait (112) est muni de moyens d'augmentation de frottement sur une surface de celui-ci qui est dirigée vers l'extérieur lorsque l'auxiliaire de retrait est dans la première position.

12. Tampon selon l'une quelconque des revendications précédentes, dans lequel le liant soluble dans un liquide est choisi parmi de l'alcool polyvinylique (PVA), de l'oxyde de polyéthylène (PEO), de la polyvinylpyrrolidone (PVP) ou des dérivés de cellulose tels que de l'hydroxypropylméthylcellulose (HPMC), de l'hydroxypropylcellulose (HPC) ou de l'hydroxyéthylcellulose (HEC).

13. Tampon selon l'une quelconque des revendications précédentes, dans lequel le tampon comprend une substance active choisie parmi des agents de régulation d'odeur, des parfums, des organismes produisant de l'acide lactique, des agents de contrôle de douleur, des sédatifs ou des mélanges de ceux-ci.
